# EUROPEAN PATENT APPLICATION

(11) **EP 4 368 714 A1**
(43) Date of publication of application: **15.05.2024**
(21) Application number: 22938949.9
(22) Date of filing: 26.04.2022
(51) Int. Cl.: C12N 15/10, C12M 1/00

(54) **SAMPLE EXTRACTION DEVICE**

(71) Applicant: Guangzhou National Laboratory, Guangzhou, Guangdong 510005 (CN)
(72) Inventor: JIANG, Taijiao, Guangzhou, Guangdong 510005 (CN); GENG, Peng, Guangzhou, Guangdong 510005 (CN); LI, Shengguang, Guangzhou, Guangdong 510005 (CN); ZHANG, Hui, Guangzhou, Guangdong 510005 (CN); LIANG, Songsong, Guangzhou, Guangdong 510005 (CN); MA, Ran, Guangzhou, Guangdong 510005 (CN); CHEN, Qixian, Guangzhou, Guangdong 510005 (CN); LIAO, Yufei, Guangzhou, Guangdong 510005 (CN)
(74) Representative: Flach Bauer & Partner Patentanwälte mbB
(86) International application number: PCT/CN2022/089372
(87) International publication number: WO 2023/206093

(57) **Abstract**

The present disclosure provides a sample extraction device, which includes a plurality of chambers arranged on a pedestal. The chamber includes a plurality of first chambers and two mixing chambers. The pedestal is internally provided with a first passage for communicating the two mixing chambers, and a sample extraction chamber is arranged in the first passage. The pedestal is further internally provided with a second passage for transfer and export of a liquid, and connection or disconnection between the two mixing chambers and the second passage is controlled by a valve. Sample extraction processes of the above technical solutions are carried out in a fully enclosed device, avoiding contamination of samples and extraction reagents by external environments, and avoiding environmental contamination caused by aerosols generated during the sample extraction processes. The sample extraction device is particularly suitable for extraction of highly pathogenic samples.

## Description

### CROSSREFERENCE TO RELATED APPLICATION

Not applicable.

### TECHNICAL FIELD

The present disclosure relates to the field of biopharmaceutical instrument technology, and more particularly, to a sample extraction device.

### BACKGROUND

At present, in biochemical laboratories individual pipettes are mainly used, to complete pipetting manually. During the extraction process, samples are exposed to the air. For transfer and detection of pathogens such as highly pathogenic and easily transmissible avian influenza virus and novel coronavirus in recent years, it is easy to increase risks of infection of the person performing the detection in an open environment. So it needs to be operated by professionals in PCR laboratories, which cannot meet the needs of large-scale detection. Although some closed sample extraction devices are reported in the prior art, gas may be generated in the process of sample extraction, so these devices also need to be provided with vent pipes or aeration basins to discharge the gas. As a result, fully sealed sample extraction cannot be really achieved. Therefore, there is an urgent need to develop a truly fully enclosed and high-throughput sample extraction device without aerosol contamination, to provide a faster and high-throughput detection method for on-site real-time detection.

### SUMMARY

To solve problems in related technologies, embodiments of the present disclosure provide a sample extraction device.

In the embodiments of the present disclosure, a sample extraction device is provided.

Specifically, the sample extraction device includes a plurality of chambers arranged on a pedestal. The chambers include a plurality of first chambers and two mixing chambers. The pedestal is internally provided with a first passage for communicating the two mixing chambers, and a sample extraction chamber is arranged in the first passage. The pedestal is further internally provided with a second passage for transfer and export of a liquid, and connection or disconnection between the two mixing chambers and the second passage is controlled by a valve.

In the embodiments of the present disclosure, a sample extraction device is also provided.

Specifically, the sample extraction device includes a plurality of chambers arranged on a pedestal, wherein the chambers include a plurality of first chambers and two mixing chambers. The plurality of first chambers include a sample suction tube, a lysis solution chamber, an empty chamber, at least one washing solution chamber, and an eluent chamber. The two mixing chambers include a first mixing chamber and a second mixing chamber. The sample suction tube, the lysis solution chamber, the empty chamber, the at least one washing solution chamber, the eluent chamber, the first mixing chamber and the second mixing chamber are sequentially arranged on the pedestal. The pedestal is internally provided with a first passage for communicating the two mixing chambers, and a sample extraction chamber is arranged in the first passage. The pedestal is further internally provided with a second passage for transfer and export of a liquid, and connection or disconnection between the two mixing chambers and the second passage is controlled by a valve.

Alternatively, the plurality of first chambers further comprise a sealant chamber and/or a purge chamber.

The sample suction tube, the lysis solution chamber, the empty chamber, the at least one washing solution chamber, the eluent chamber, the sealant chamber, the first mixing chamber, the second mixing chamber, and the purge chamber are sequentially arranged on the pedestal.

Alternatively, the sample suction tube, the lysis solution chamber, the empty chamber, the at least one washing solution chamber, the eluent chamber, the first mixing chamber, the second mixing chamber, and the purge chamber are sequentially arranged on the pedestal.

Alternatively, the sample suction tube, the lysis solution chamber, the empty chamber, the at least one washing solution chamber, the eluent chamber, the sealant chamber, the first mixing chamber and the second mixing chamber are sequentially arranged on the pedestal.

Alternatively, the plurality of first chambers further comprise a sealant chamber and/or a purge chamber.

The sample suction tube, the lysis solution chamber, the empty chamber, the at least one washing solution chamber, the first mixing chamber, the second mixing chamber, the sealant chamber, the eluent chamber, and the purge chamber are sequentially arranged on the pedestal.

Alternatively, the sample suction tube, the lysis solution chamber, the empty chamber, the at least one washing solution chamber, the first mixing chamber, the second mixing chamber, the sealant chamber, and the eluent chamber are sequentially arranged on the pedestal.

Alternatively, the sample suction tube, the lysis solution chamber, the empty chamber, the at least one washing solution chamber, the first mixing chamber, the second mixing chamber, the eluent chamber, and the purge chamber are sequentially arranged on the pedestal.

Alternatively, the sample extraction device further comprises a valve assembly configured to control connection or disconnection between the second passage and the plurality of first chambers.

Alternatively, at least one of the plurality of first chambers is preloaded with a reagent required for sample extraction.

Alternatively, the plurality of chambers are sealed by a sealing element, which is selected from a membrane, a piston, or a piston rod.

Alternatively, the two mixing chambers are arranged with a preset interval; and/or, a second cavity is provided below the sample extraction chamber.

Alternatively, a magnetic bead reagent is prepackaged in the sample extraction chamber.

Alternatively, the sample extraction device also includes: a quantitation chamber positioned within the second passage.

Alternatively, the valve assembly is consisted of a plurality of valves; and the second passage has branches extending towards the first chambers and the mixing chambers, and connection or disconnection between each of the branches and a first chamber or a mixing chamber is independently controlled by one of the plurality of valves.

Alternatively, the valve is a rotating member, and the plurality of valves are arranged linearly at a bottom of the pedestal.

Alternatively, the bottom of the pedestal is provided with a plurality of first cavities, and each of the plurality of first cavities is provided with a valve having an adapter channel. When the valve is placed in the first cavity, one end of the adapter channel is communicated with a chamber, and another end of the adapter channel is communicated with a second passage under an external force.

Alternatively, the sample extraction device also includes a sealing plug arranged in the first cavity, where the sealing plug is respectively communicated with a chamber and a second passage, and the adapter channel is placed in the sealing plug.

Alternatively, the sealing plug is a column structure, a through hole is provided in a center of the sealing plug, and at least one open hole is provided on a side wall of the sealing plug.

Alternatively, the valve comprises a valve handle and a valve shaft, where the valve handle is movably connected to the pedestal, and the adapter channel is arranged inside the valve shaft.

Alternatively, the valve handle and valve shaft are of an integrated or detachable structure.

Alternatively, one end of the valve handle is provided with a limiting slot.

Alternatively, the sample extraction device also includes: a first sample loading tube and a first sample loading runner arranged on the pedestal, wherein the first sample loading runner is controlled by a valve to connect the first sample loading tube to one of the first chambers, and/or one of the first chambers is internally provided with a first push-pull piston rod.

Alternatively, a branch tube is communicated with at least one of the first chambers, and/or the at least one of the first chambers is internally provided with a third push-pull piston rod, wherein a top of the third push-pull piston rod is provided with a sealing ring. When the third push-pull piston rod is pushed to a bottom, the sealing ring is positioned above the branch tube.

Alternatively, the at least one of the first chamber is a sample suction tube.

Alternatively, the second passage includes a liquid outlet channel.

The sample extraction device further comprises: a second sample loading tube, a second sample loading runner and a sample collection tube arranged on the pedestal; wherein the sample collection tube is arranged at a liquid outlet of the liquid outlet channel.

The second sample loading runner is controlled by a valve to connect the second sample loading tube to the liquid outlet channel, wherein the second sample loading tube has a branch tube.

Alternatively, the second sample loading tube is internally provided with a third push-pull piston rod, wherein a top of the third push-pull piston rod is provided with a sealing ring. When the third push-pull piston rod is pushed to a bottom, the sealing ring is positioned above the branch tube.

Alternatively, the sample extraction device also includes: a sample collection tube arranged on the pedestal; and a gas exhaust channel controlled by a valve to connect one of the plurality of first chambers to the sample collection tube.

Alternatively, the pedestal includes a pedestal body and an outer protective plate positioned on a front face and a back face of the pedestal body, wherein at least one groove is provided on the front face and/or back face of the pedestal body; the groove and the outer protective plate form the first passage and the second passage.

Alternatively, the sample extraction device also includes: a sample collection tube arranged on the pedestal.

The second passage includes: a first channel controlled by a valve respectively to connect the at least one washing solution chamber, the eluent chamber, and the first mixing chamber; a second channel controlled by a valve respectively to connect the sample suction tube, the lysis solution chamber, the empty chamber, and the first mixing chamber; a sample quantification channel controlled by a valve respectively to connect the sealant chamber and/or the purge chamber and the second mixing chamber, wherein the sample quantification channel is internally provided with a quantification chamber; a liquid outlet channel controlled by a valve respectively to connect the sample quantification channel and the sample collection tube; and a gas exhaust channel controlled by a valve to connect the eluent chamber and the sample collection tube.

Alternatively, the sample extraction device also includes: a sample collection tube arranged on the pedestal.

The second passage includes: a third channel controlled by a valve respectively to connect the at least one washing solution chamber and the first mixing chamber; a fourth channel controlled by a valve respectively to connect the eluent chamber and the second mixing chamber; a fifth channel controlled by a valve respectively to connect the sample suction tube, the lysis solution chamber, the empty chamber, and the first mixing chamber; a sample quantification channel controlled by a valve respectively to connect the second mixing chamber, the sealant chamber and/or the purge chamber, wherein the sample quantification channel is internally provided with a quantification chamber; a liquid outlet channel controlled by a valve respectively to connect the sample quantification channel and the sample collection tube; and a gas exhaust channel controlled by a valve to connect the purge chamber and the sample collection tube.

According to the technical solutions provided by the above embodiments of the present disclosure, the technical solutions of the present disclosure can achieve following beneficial effects.

For the sample extraction device of the present disclosure, sample extraction processes are carried out in a fully enclosed device, avoiding contamination of samples and extraction reagents by external environments, and avoiding environmental contamination caused by aerosols generated during the sample extraction processes. The sample extraction device of the present disclosure is particularly suitable for extraction of highly pathogenic samples. Connection or disconnection of the sample, the extraction reagent, and the second passage may be controlled separately, such that transfer of the sample and the extraction reagent may be carried out separately, which avoids possible cross contamination between the sample and the reagent or between different reagents, and thus avoiding affecting accuracy of subsequent sample detection.

For the sample extraction device of the present disclosure, connection or disconnection between each first chamber, each mixing chamber, and the second passage is independently controlled by rotating the valve, facilitating adaptation of mechanical driving devices. Furthermore, a plurality of sample extraction devices may be connected in parallel for use, such that a plurality of samples may be automatically detected at one go, thereby improving detection efficiency.

For the sample extraction device of the present disclosure, magnetic beads are adsorbed by a permanent magnet and are located within the sample extraction chamber without transfer. The samples and the reagents are transferred to the sample extraction chamber, where the magnetic beads are used to adsorb nucleic acid obtained after pyrolysis of the samples, and then the nucleic acid is eluted with the reagents, thereby achieving extraction of the nucleic acid. Compared with the existing technologies where the nucleic acid is extracted by means of transfer of the magnetic beads, the sample extraction device can avoid potential loss of magnetic beads or blockage of runners caused by the transfer of the magnetic beads, thus improving efficiency of sample extraction.

For the sample extraction device of the present disclosure, two mixing chambers are connected to achieve uniform and sufficient mixing of the samples and the extraction reagents, thereby improving the efficiency of sample extraction.

For the sample extraction device of the present disclosure, a quantitation chamber is provided in the second passage, which can achieve quantitative extraction of the samples. A gas exhaust channel is provided to discharge gas from the sample collection tube into one of the chambers, avoiding failure of the extracted samples entering the sample collection tube due to excessive air pressure of the sample collection tube. The samples transferred to the sample collection tube can be directly tested, thereby achieving integration of sample extraction and detection, and thus improving the efficiency of sample detection.

It is to be understood that the above general description and the detailed description below are merely exemplary and explanatory, and do not limit the present disclosure.

### BRIEF DESCRIPTION OF THE DRAWINGS

To describe the technical solutions of the embodiments of the present disclosure or those of the related technologies more clearly, the accompanying drawings required for describing the exemplary embodiments or the related technologies will be briefly introduced below. Obviously, the accompanying drawings in the following description are merely some exemplary embodiments of the present disclosure. To those of ordinary skills in the art, other accompanying drawings may also be derived from these accompanying drawings without creative efforts.
FIG. 1 shows a main view of a sample extraction device of the present disclosure.
FIG. 2 shows an exploded view of a sample extraction device of the present disclosure.
FIG. 3 shows a schematic diagram of A-side and B-side of a pedestal body of the present disclosure.
FIG. 4 shows a perspective view of A-side of a sample extraction device of the present disclosure.
FIG. 5 shows a perspective view of B-side of a sample extraction device of the present disclosure.
FIG. 6 shows a schematic structural diagram of a rubber plug of the present disclosure.
FIG. 7 shows a schematic structural diagram of a valve of the present disclosure.
FIG. 8 shows a main view of a further sample extraction device of the present disclosure.
FIG. 9 shows an exploded view of a further sample extraction device of the present disclosure.
FIG. 10 shows a schematic diagram of A-side and B-side of a furtherpedestal body of the present disclosure.
FIG. 11 shows a perspective view of A-side of a further sample extraction device of the present disclosure.
FIG. 12 shows a perspective view of B-side of a further sample extraction device of the present disclosure.

### DETAILED DESCRIPTION

In the following text, the exemplary embodiments of the present disclosure will be described in detail with reference to the accompanying drawings, such that those skilled in the art can easily implement them. Furthermore, for clarity, parts unrelated to describing the exemplary embodiments have been omitted from the accompanying drawings.

In the present disclosure, it should be understood that terms such as "including" or "having" are intended to indicate the existence of the features, numbers, steps, behaviors, components, parts or combinations thereof in this specification, and are not intended to exclude the possibility of one or more other features, numbers, steps, behaviors, components, parts or combinations thereof being present or added.

The same or similar reference numerals in the accompanying drawings of the present disclosure correspond to the same or similar components. In the description of the present disclosure, it should be understood that the orientation or position relations represented by the terms such as "center", "up", "down", "left", "right", "horizontal", "inside", "outside" and the like are orientation or position relations shown based on the accompanying drawings, they are merely for ease of a description of the present disclosure and a simplified description instead of being intended to indicate or imply the device or element to have a special orientation or to be configured and operated in a special orientation. Thus, they cannot be understood as limiting of the present disclosure. For those ordinarily skilled in the art, specific meanings of the aforementioned terms may be understood based on specific circumstances. In addition, terms such as "first", "second" and "third" are merely for discriminative description of the components, and cannot be construed as indicating or implying the relative importance.

In the description of the present disclosure, it is to be noted that unless specified or limited otherwise, terms "arrangement", "installation", "connecting" or "connection" should be understood in a broad sense, which may be, for example, a fixed connection, a detachable connection or integrated connection; a mechanical connection or an electrical connection; a direct connection or indirect connection by means of an intermediary, or internal communication between two elements. For those of ordinary skill in the art, concrete meanings of the above terms in the present disclosure may be understood based on concrete circumstances.

In addition, it is to be noted that the embodiments of the present disclosure and the features in the embodiments may be combined with each other with the proviso that there is no conflict. The present disclosure will be described below in details with reference to the accompanying drawings and in combination with the examples.

### Example 1

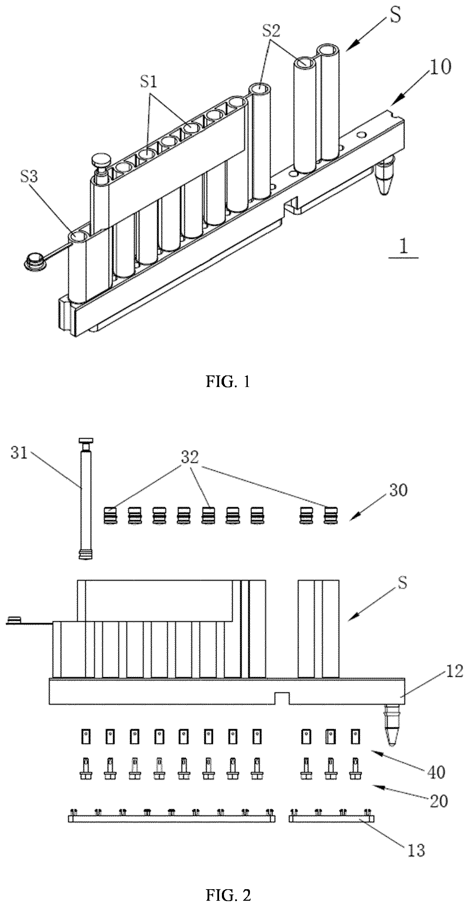

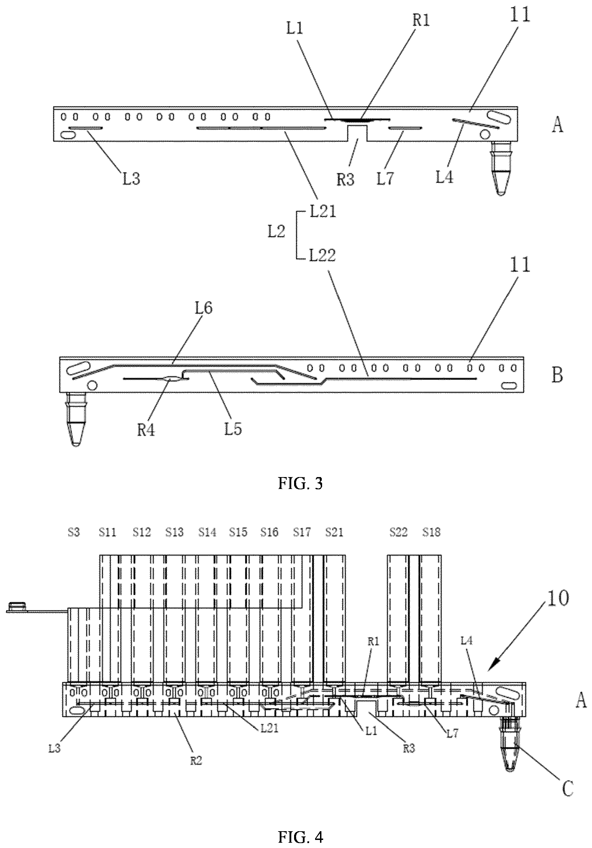

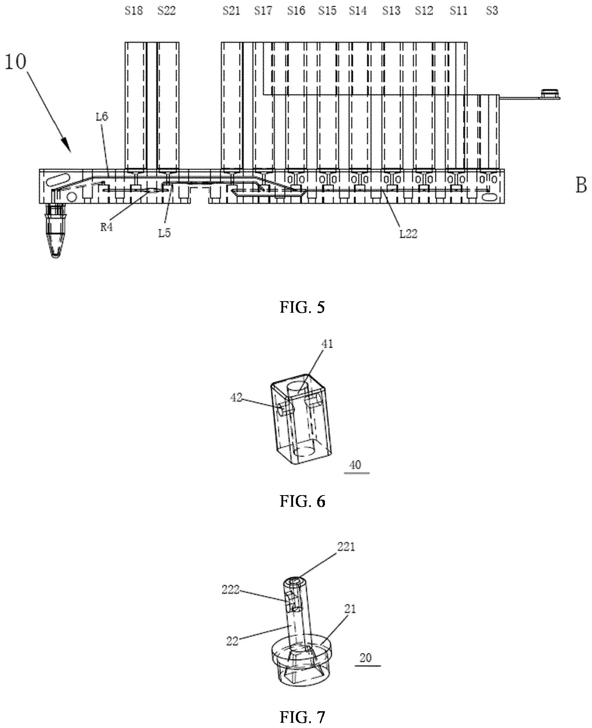

FIG. 1 shows a main view of a sample extraction device of the present disclosure. FIG. 2 shows an exploded view of a sample extraction device of the present disclosure. FIG. 3 shows a schematic diagram of A-side and B-side of a pedestal body of the present disclosure. FIG. 4 shows a perspective view of A-side of a sample extraction device of the present disclosure. FIG. 5 shows a perspective view of B-side of a sample extraction device of the present disclosure.

As shown in FIGs. 1 to 5, a sample extraction device 1 includes a pedestal 10 and a plurality of chambers S arranged on the pedestal 10. The pedestal 10 is internally provided with a first passage L1 and a second passage L2. The first passage L1 and the second passage L2 are pipelines for passage of liquids, gases, and other fluids. The second passage L2 is used for transfer and export of liquids such as samples and reagents. Specifically, the pedestal 10 includes a pedestal body 11 and an outer protective plate 12 positioned on the A-side and the B-side of the pedestal body 11 (FIG. 2 only shows the outer protective plate of the A-side), wherein the A-side and the B-side of the pedestal body 11 are respectively provided with at least one groove. The outer protective plate 12 covers the A-side and the B-side of the pedestal body 11, and the outer protective plate 12 forms, together with at least one groove, the first passage L1, at least one second passage L2, and a liquid outlet channel L4. In the following explanation, the A-side of the pedestal body 11 is also referred to as a front side of the pedestal 10, and the B-side of the pedestal body 11 is also referred to as a back side of the pedestal 10.

Chambers S include a plurality of first chambers S1 and two mixing chambers S2 such as a first mixing chamber S21 and a second mixing chamber S22 (referring to FIG. 4 and FIG. 5). The two mixing chambers S2 are connected by the first passage L1, which is internally provided with a sample extraction chamber R1. Connection or disconnection between the two mixing chambers S2 and the second passage L2 is also controlled by a valve 20. Specifically, each mixing chamber S2 may be adapted to one valve 20 or two mixing chambers S2 may share one valve 20, which is not limited by the present disclosure. In addition, connection or disconnection between the two mixing chambers S2 and the liquid outlet channel L4 is also controlled by the valve 20.

The second passage L2 is also provided with a valve assembly, which controls the connection or disconnection between the plurality of first chambers S1 and the second passage L2. The second passage L2 has branches extending towards the first chambers S1 and the mixing chambers S2. The valve assembly may be comprised of a plurality of valves 20, each of which controls the connection or disconnection between one of the first chambers S1 and one of the branches. Different branches are independent of each other, thus avoiding liquid mixing between different chambers during transfer of liquids in the chambers S, thereby avoiding contamination. Of course, the valve assembly may also be a rotating member with a plurality of channels, and each branch is connected to a different channel by rotation of the valve assembly, thereby avoiding mixing the liquid. The present disclosure is not limited to a specific form of the valve assembly.

When the sample extraction device of the present disclosure is used, a sample and an extraction reagent are provided in the plurality of chambers , and an external driving component is placed in the chambers. Through cooperation between the external driving component, the valves and the valve assembly, the sample and the extraction reagent are transferred to the two mixing chambers through the second passage. The two mixing chambers are connected to the sample extraction chamber, such that the sample and the extraction reagent can be mixed in the two mixing chambers again and again, and the extraction is completed in the sample extraction chamber. Finally, the sample and the extraction reagent are transferred out through the liquid outlet channel. The entire process is carried out inside the enclosed device, thus avoiding contamination of the samples and the extraction reagents by external environments. Connection or disconnection of the sample, the extraction reagent and the second passage may be controlled separately, such that transfer of the sample and the extraction reagent may be carried out separately, which avoids possible cross contamination between the sample and the reagent or different reagents, and thus avoiding affecting accuracy of subsequent sample detection. The sample extraction device of the present disclosure is particularly suitable for nucleic acid extraction, which can avoid possible contamination in the process of nucleic acid extraction and improve accuracy of nucleic acid detection. It is to be understood that the sample extraction device of the present disclosure is not limited to nucleic acid extraction, but also may be applied to extraction of other samples demanding for environmental requirements, and it does not constitute a limitation of the present disclosure.

According to the embodiments of the present disclosure, at least one of the plurality of first chambers S1 may be preloaded with a reagent required for sample extraction, and may be sealed by a sealing element, which is selected from a membrane, a piston, or a piston rod such as a push-pull piston rod 30, to avoid contamination of the extracted reagent when exposed to the external environment or to avoid contamination of the sample and the reagent caused by the first chamber S1 during the process of placing an external driving component, thereby improving the accuracy of sample detection.

According to the embodiments of the present disclosure, on/off of the valves 20 may be controlled by means of electromagnetic driving, mechanical driving, and the like. In the present disclosure, a mechanical driving means is provided. Specifically, the valve 20 is a rotating member, and the valves that are each adapted to a first chamber S1 or a mixing chamber S2 are arranged at the bottom of pedestal 10 linearly . By rotating the valve 20, connection or disconnection between each first chamber S1, or each mixing chamber S2, and a branch of the second passage L2 is independently controlled. Therefore, the sample extraction device of the present disclosure may be adapted to a mechanical driving device. The plurality of valves 20 are arranged in a linear manner and thus may be adapted to a equal number of linear motors, and the linear motors separately drive the valves 20 to control their connection or disconnection. When a plurality of sample extraction devices are connected in parallel, multi-paralleled valves 20 are formed, which facilitates simultaneously controlling on/off of each of the valves 20 by means of mechanical driving, such that a plurality of samples can be automatically detected at one go, thereby improving detection efficiency.

In the present disclosure, a plurality of first cavities R2 are provided on the bottom of the pedestal 10, where each of the first cavities R2 is internally provided with one valve 20.

In the present disclosure, the valve 20 has an adapter channel. When the valve 20 is placed in the first cavity R2, one end of the adapter channel is communicated with the chamber S, and other end of the adapter channel is communicated with the second passage L2 under an external force.

According to the embodiments of the present disclosure, referring to FIG. 6, the sample extraction device 1 also includes a sealing plug 40 arranged in the first cavity R2, wherein the sealing plug 40 is respectively communicated with the chamber S and the second passage L2, and the adapter channel is placed in the sealing plug 40. The sealing plug 40 may be a rubber plug or a polyurethane plug, and a shape of the sealing plug 40 is adapted to the first cavity R2. For example, the sealing plug 40 is a column structure, such that the sealing plug 40 does not rotate with respect to the first cavity R2, thus avoiding blocking a passage between the chamber S and the second passage L2. Of course, other adapted shapes may also be used, and the present disclosure is not limit thereto. By providing the sealing plug 40 such that rotation of the valve 20 occurs within the sealing plug 40, it can prevent leakage of a liquid when the liquid is transferred from chamber S to the second passage L2, which reduces possible contamination, and thus further improves accuracy of sample detection.

FIG. 6 shows a schematic structural diagram of a rubber plug of the present disclosure. As shown in FIG. 6, the sealing plug 40 is a column structure, a through hole 41 is provided in a center of the sealing plug 40, and at least one first open hole 42 is provided on a side wall of the sealing plug 40. The through hole 41 is communicated with the chamber S, and number of the first open holes 42 matches that of branches of the second passage L2. For example, for one first chamber S1, when the front face and the back face of the pedestal 10 are respectively provided with a second passage L2, the number of the first open holes 42 should be two to achieve separate communication with the second passage L2.

FIG. 7 shows a schematic structural diagram of a valve of the present disclosure. As shown in FIG. 7, the valve 20 includes a valve handle 21 and a valve shaft 22. A second open hole 221 may be provided at a center of a top of the valve shaft 22, and a valve hole 222 may be machined and formed on a side wall near the top of the valve shaft 22. For example, the valve hole 222 may be a circular or waist-type blind hole. Structures of the second open hole 221 and the valve hole 222 form the adapter channel. In the present disclosure, number of the second open hole 222 is one, and the second open hole 222 forms, with the sealing plug 40, a two-way valve or a three-way valve.

Specifically, referring to FIG. 2, during assembly, the rubber plug 40 is first placed into the first cavity R2, and then the valve shaft 22 is placed into the through hole 41. The valve handle 21 is limited by a valve shaft pressure plate 13, and next the valve shaft pressure plate 13 is fixed to the bottom of the pedestal body 11. The valve handle 21 can rotate with respect to the valve shaft pressure plate 13. When the number of the first open holes 42 is two, the valve 20 and the sealing plug 40 form one three-way valve. Under an acting force of the mechanical driving, a relative position between the valve hole 222 and the first open hole 42 may be adjusted to selectively communicate with one of the first open holes 42, thereby achieving the communication between the chamber S and the second passage L2, and achieving the blocking between the chamber S and the second passage L2 when the positions of the valve hole 222 and the first open hole 42 are misaligned.

The valve handle 21 and the valve shaft 22 are of an integrated or detachable structure. For example, the valve handle 21 and the valve shaft 22 may be formed by means of integrated molding, or the valve handle 21 and the valve shaft 22 may be formed separately, and then are connected and fixed by means of threaded connections or the like, but the present disclosure is not limited thereto. An end face of the valve handle 21 may be machined to form a limiting slot such as alinear slot, a cross-shaped slot, or a slot with other shapes, to adapt to the mechanical driving device as mentioned above, which is not described herein again.

According to the embodiments of the present disclosure, the sample extraction device 1 further comprises a first sample loading tube S3 and a sample loading runner L3 arranged on the pedestal 10, wherein the sample loading runner L3 is controlled by one valve 20 to connect the first sample loading tube S3 to one of the first chambers S1. In the present disclosure, the reagent is prepackaged in the chamber S, and the sample is injected from the first sample loading tube S3 after sampling. Next, the first sample loading tube S3 is covered with a lid, thereby forming a sealed environment for the sample extraction device 1 as a whole. The sample loading runner L3 is communicated with one of the first chambers S1 under the control of the valve 20. Next, the sample is transferred through the sample loading runner L3 to the first chamber S1 by a negative pressure formed by a pumping action of the external driving component or the push-pull piston rod 30 placed in chamber S. Next, transfer of the sample and the reagent is achieved through the second passage L2.

The sample extraction device of the present disclosure is described in details below through one specific example.

The sample extraction device of the present disclosure is suitable for nucleic acid extraction by a magnetic beads method. Here, it is necessary to first explain principles of magnetic beads method for nucleic acid extraction. After the sample is injected into a lysis solution, the nucleic acid is released, and then the treated magnetic beads (such as coated with silicon or amino groups) are used to carry out "specific binding" with the nucleic acid to form a "nucleic acid-magnetic bead complex". Next, under the action of an external magnetic field, the complex is isolated, and finally the nucleic acid substance to be extracted is obtained by washing away non-specifically absorptive impurities with an eluent, followed by desalting and purifying.

As shown in FIG. 4 and FIG. 5, the sample extraction device of the present disclosure comprises a plurality of chambers S arranged on the pedestal 10, where the chambers S comprise a plurality of first chambers S1 and two mixing chambers S2. The plurality of first chambers S1 comprise a sample suction tube S11, a lysis solution chamber S12, an empty chamber S13, at least one washing solution chamber such as a first washing solution chamber S14 and a second washing solution chamber S15, and an eluent chamber S16. The two mixing chambers S2 comprise a first mixing chamber S21 and a second mixing chamber S22. The sample suction tube S11, the lysis solution chamber S12, the empty chamber S13, the at least one washing solution chamber, the eluent chamber S16, the first mixing chamber S21 and the second mixing chamber S22 are sequentially arranged on the pedestal 10. The pedestal 10 is internally provided with a first passage L1 for communicating the two mixing chambers S2, and a sample extraction chamber R1 is arranged in the first passage L1. The pedestal 10 is further internally provided with a second passage L2 for transfer and export of a liquid. Connection or disconnection between the two mixing chambers S2 and the second passage L2 is controlled by a valve 20.

As a preferred solution, the sample extraction device of the present disclosure further includes a sealant chamber S17, which is internally provided with a sealing reagent selected from at least one of mineral oil, silicone oil, fluoroalkane oil, vegetable oil, and liquid paraffin. The sample suction tube S11, the lysis solution chamber S12, the empty chamber S13, the at least one washing solution chamber, the eluent chamber S16, the sealant chamber S17, the first mixing chamber S21, and the second mixing chamber S22 are sequentially arranged on the pedestal 10.

As another preferred solution, the sample extraction device of the present disclosure also includes a purge chamber S18. The sample suction tube S11, the lysis solution chamber S12, the empty chamber S13, the at least one washing solution chamber, the eluent chamber S16, the first mixing chamber S21, the second mixing chamber S22, and the purge chamber S18 are sequentially arranged on the pedestal 10.

As another preferred solution, the sample extraction device of the present disclosure further includes a sealant chamber S17 and a purge chamber S18. The sample suction tube S11, the lysis solution chamber S12, the empty chamber S13, the at least one washing solution chamber, the eluent chamber S16, the sealant chamber S17, the first mixing chamber S21, the second mixing chamber S22, and the purge chamber S18 are sequentially arranged on the pedestal 10.

In the present disclosure, the sample extraction device further comprises a push-pull piston rod 30. There are two types of the push-pull piston rod 30. One is a longer first push-pull piston rod 31 placed in the sample suction tube 11. A length of the first push-pull piston rod 31 is greater than that of the sample suction tube S11. The first push-pull piston rod 31 is configured to suck the sample in the first sample loading tube S3 into the sample suction tube S11. The other one is a shorter second push-pull piston rod 32 placed in the lysis solution chamber S12, the empty chamber S13, the first washing solution chamber S14, the second washing solution chamber S15, the eluent chamber S16, the sealant chamber S17, the purge chamber S18, the first mixing chamber S21 and the second mixing chamber S22, and plays a role in sealing and driving the transfer of the reagent.

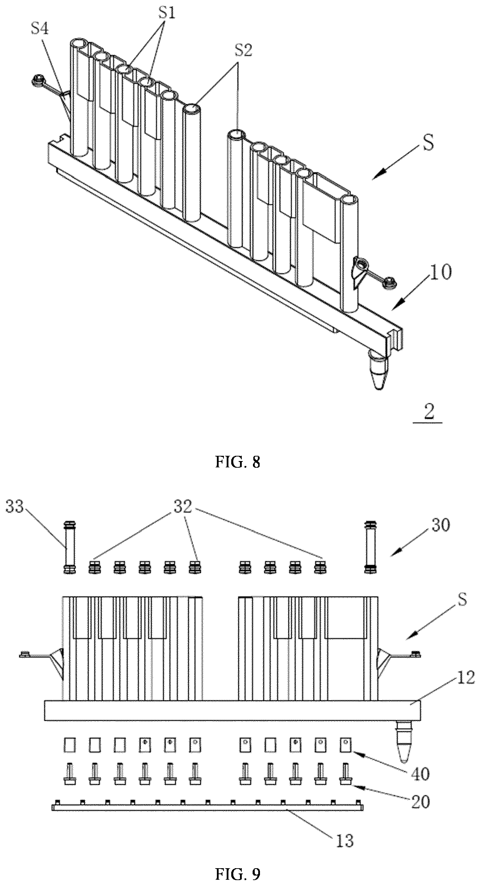

In the present disclosure, at least one side wall of the plurality of first chambers S1 may be provided with a branch tube S4 to inject the sample or the extraction reagent into the chamber S1. For example, the side wall of the sample suction tube S11 is provided with the branch tube S4, through which the sample can be injected, thereby omitting the sample loading runner L3 connecting the first sample loading tube S3 and the first chamber S1. Correspondingly, the sample suction tube S11 is internally provided with a third push-pull piston rod 33 (referring to FIG. 9), wherein a top of the third push-pull piston rod 33 is provided with a sealing ring. When the third push-pull piston rod 33 is pushed to a bottom, the sealing ring is positioned above the branch tube S4 to prevent leakage of the reagent from the branch tube S4 to the external environment.

Please also refer to FIG. 3, the front face of the pedestal 10 is provided with a first passage L1, a first channel L21, a sample loading runner L3, a liquid outlet channel L4, and a washing channel L7. The back face of the pedestal 10 is provided with a second channel L22, a sample quantification channel L5, and a gas exhaust channel L6.

The first passage L1 connects the first mixing chamber S21 to the second mixing chamber S22. The first passage L1 is internally provided with a sample extraction chamber R1, which is prepackaged with a magnetic bead reagent for adsorbing the nucleic acid in the sample. The first mixing chamber S21 and the second mixing chamber S22 are arranged with a preset interval to attach an external heating device. A second cavity R3 is arranged below the sample extraction chamber R1, and a permanent magnet may be placed in the second cavity R3 to adsorb the magnetic beads.

The first channel L21 is controlled by the valve 20 respectively to connect the first washing solution chamber S14, the second washing solution chamber S15, the eluent chamber S16, and the first mixing chamber S21. The second channel L22 is controlled by the valve 20 respectively to connect the sample suction tube S11, the lysis solution chamber S12, the empty chamber S13, and the first mixing chamber S21.

The sample loading runner L3 is controlled by the valve 20 to connect the first sample loading tube S3 and the sample suction tube S11.

The liquid outlet channel L4 is controlled by the valve 20 to connect the sample collection tube (indicated as C in the figures) to the sample quantification channel L5.

The sample quantification channel L5 is controlled by the valve 20 respectively to connect the sealant chamber S17 and/or the purge chamber S18 and the second mixing chamber S22. The sample quantification channel L5 is internally provided with a quantification chamber R4, which is positioned in a passage part between the second mixing chamber S22 and the purge chamber S 18 connected under the control of the valve 20.

The gas exhaust channel L6 is connected to the eluent chamber S16 and the sample collection tube under the control of the valve 20.

The washing channel L7 is controlled by the valve 20 to connect the passage part between the second mixing chamber S22 and the purge chamber S18.

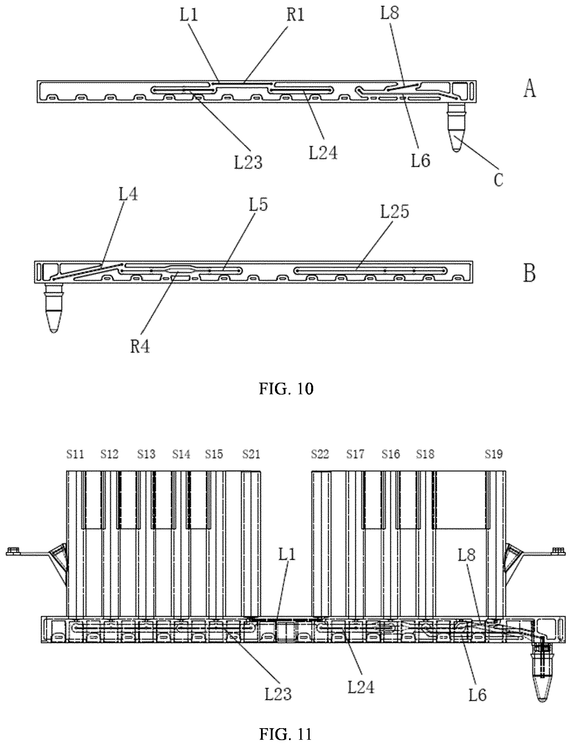

In the present disclosure, as shown in FIG. 11, a second sample loading tube S19 may also be arranged on the pedestal 10, wherein the second sample loading tube S19 also uses a branch tube S4 to load the sample, and correspondingly a second sample loading runner L8 is arranged. Communication between the second sample loading runner L8 and a liquid outlet runner L4 is achieved through a valve 20, wherein the liquid outlet runner L4 is directly connected to the sample collection tube (indicated as C in the figures), such that a secondary reagent may be directly loaded to the sample collection tube. In this way, the secondary reagent may be loaded again after sample extraction, which expands range of application of the sample extraction device. (Generally the secondary reagent is a reagent not suitable for direct transfer in the sample extraction device, such as a reagent that requires for low-temperature preservation. The present disclosure does not limit the secondary reagent that can be loaded). In the present disclosure, steps for nucleic acid extraction using the sample extraction device are as follows.

In Step S101 of preprocessing, prior to the detection, each valve is maintained in an initialization position, such that all the channels are in an off state. The reagents, the magnetic beads and the like required for nucleic acid extraction are prepackaged in corresponding chambers, and a push-pull piston rod is placed in each chamber for sealing.

For example, 750ul of a lysis solution is prepackaged in the lysis solution chamber S12, 700ul of a washing solution is respectively prepackaged in the first washing solution chamber S14 and the second washing solution chamber S15, 100ul of an eluant is prepackaged in the eluent chamber S16, 50ul of a mineral oil is prepackaged in the sealing reagent chamber S17, 600ul of a magnetic bead solution is prepackaged in the first mixing chamber S21, and 600ul of ultrapure water or pure air is prepackaged in the purge chamber S18.

In Step S102 of sample loading, a lid of the first sample loading tube S3 is opened, the collected sample is input into the sample loading tube by means of a pipette, and then the lid is covered.

In Step S103 of quantitative sample suction, the push-pull piston rod in the sample suction tube S11 is driven to move upwards, and the liquid sample (such as 200ul of nucleic acid sample) in the first sample loading tube S3 is quantitatively introduced into the sample suction tube S11 by means of negative pressure.

In Step S104 of nucleic acid lysis, the push-pull piston rods in the first mixing chamber S21 and the second mixing chamber S22 are driven to move, such that the magnetic bead solution flows back and forth between the two mixing chambers. Next, the magnetic beads are adsorbed in the sample extraction chamber R1 by means of a magnet, and waste liquid is discharged into the empty chamber S13. Afterwards, the valves corresponding to the sample suction tube S11 and the first mixing chamber S21 are opened to transfer the sample to the first mixing chamber S21, the valve corresponding to the sample suction tube S11 is closed, and the valve corresponding to the lysis solution chamber S12 is opened to transfer the lysis solution to the first mixing chamber S21. Next, the push-pull piston rods in the first mixing chamber S21 and the second mixing chamber S22 are driven to move, such that a mixed liquor of the sample and the lysis solution is mixed, heated and decomposed in the sample extraction chamber R1; and decomposed nucleic acid molecules may be adsorbed by the magnetic beads in the sample extraction chamber R1. Next, the waste liquid is discharged back to the lysis solution chamber S12.

In Step S105 of cleaning the nucleic acid, by using a method similar to Step S104, the valves corresponding to the first washing solution chamber S14, the second washing solution chamber S15 and the eluent chamber S16 are respectively opened, such that the liquid reagent in each chamber enters the first mixing chamber S21 and the second mixing chamber S22 for mixing, adsorption, cleaning, and elution. The cleaned or eluted waste liquid is discharged into the original chambers for storage.

In Step S106 of quantitation: the valves (the valves respectively corresponding to the second mixing chamber S22 and the purge chamber S18) corresponding to two ends of the quantitation chamber R4 are opened respectively, the other valves are remained off, and the samples in the first mixing chamber S21 and the second mixing chamber S22 are injected into the quantitation chamber R4 (the quantitative volume may be 5ul) such as in a diamond-shaped area. To ensure that the quantitation chamber R4 is filled with liquid and to ensure quantitation accuracy, in the present disclosure, a portion of the liquid may be injected into the purge chamber S18.

In Step S107 of transferring the nucleic acid, the valves corresponding to the second mixing chamber S22 and the purge chamber S18 are closed, the valve corresponding to the sealant chamber S17 and the valve for controlling connection of the liquid outlet channel L4 are opened, the push-pull piston rod in the sealant chamber S17 is controlled to move downwards, and the sample is quantitatively discharged into the sample collection tube using the mineral oil, wherein the mineral oil may also be used as the reagent finally required for covering. To avoid a positive pressure of the sample collection tube adversely affecting export of the sample, the sample collection tube and the eluent chamber may be connected before the export of the sample, and gas in the sample collection tube may be discharged into the eluent chamber by means of the gas exhaust channel. It is to be understood that the gas exhaust channel may also be connected to other chambers such as the empty chamber, but the present disclosure is not limited thereto.

The above steps may also include a pipeline cleaning step. For example, before transferring different liquids from the same pipeline, the ultrapure water or pure air prepackaged in the purge chamber S18 may be used to clean the pipeline, and then the liquids are transferred. In this way, cross impact of the reagents can be avoided. However, this step is not necessary and may be flexibly selected as needed. It is to be noted that, similar to the cleaning step, the quantitation step S106 is also an optional step. Those skilled in the art can flexibly choose according to needs, such as omitting or retaining corresponding passages and chambers, which is not limited in the present disclosure.

### Example 2

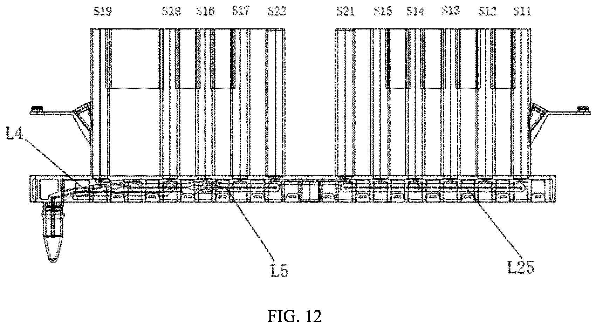

FIG. 8 shows a main view of a further sample extraction device of the present disclosure. FIG. 9 shows an exploded view of a further sample extraction device of the present disclosure. FIG. 10 shows a schematic diagram of A-side and B-side of a further pedestal body of the present disclosure. FIG. 11 shows a perspective view of A-side of a further sample extraction device of the present disclosure. FIG. 12 shows a perspective view of B-side of a further sample extraction device of the present disclosure.

As shown in FIG. 8 and FIG. 9, a difference between the sample extraction device 2 provided in the present disclosure and the sample extraction device as shown in FIGs. 1 to 5 lies in that the first sample loading tube S3 and the sample loading runner L3 are not used when the sample is loaded. Instead one of the first chambers S1 is used as the sample suction tube. Specifically, a branch tube S4 is arranged on the side wall of the first chamber S1, and the sample is loaded through the branch tube S4, such that the sample loading runner L3 connecting the first sample loading tube S3 and the first chamber S1 may be omitted. After the sample is added into the first chamber S1 through the branch tube S4, the branch tube S4 is covered with a lid thereon to form a sealed environment for the sample extraction device 1 as a whole. Next, the corresponding valve 20 is opened, and then the sample is transferred to the mixing chamber S2 through a pushing force of the external driving component or the push-pull piston rod 30 placed in chamber S. As shown in FIG. 2 and FIG. 9, the push-pull piston rods 30 have different lengths. When using the first sample loading tube S3 for sample loading, the longer first push-pull piston rod 31 is used to provide the suction force. When using the branch tube S4 for sample loading, the shorter third push-pull piston rod 33 is used to provide the pushing force. In some cases, in addition to sample loading through a branch tube S4, other first chambers S1 may also be provided with a branch tube S4 for loading the reagent, but the present disclosure is not limited thereto.

Further, for the first chamber S1 without the use of the branch tube S4, the shorter second push-pull piston rod 32 may be used and placed in the corresponding first chamber S1 to seal and drive transfer of the reagent. For example, a second push-pull piston rod 32 is positioned inside the lysis solution chamber S12, the empty chamber S13, the first washing solution chamber S14, the second washing solution chamber S15, the first mixing chamber S21, the second mixing chamber S22, the eluent chamber S16, the sealant chamber S17, and the purge chamber S18, referring to FIG. 11 and FIG. 12.

As shown in FIG. 11 and FIG. 12, the difference between the sample extraction device 2 provided in the present disclosure and the sample extraction device shown in FIGs. 1 to 5 also lies in that a second sample loading tube S19 is also arranged on the other end of the pedestal 10, wherein the second sample loading tube S19 also uses a branch tube S4 to load the sample, and correspondingly a second sample loading runner L8 is arranged. Communication between the second sample loading runner L8 and a liquid outlet runner L4 is achieved through a valve 20, where the liquid outlet runner L4 is directly connected to the sample collection tube (indicated as C in the figures), such that a secondary reagent may be directly loaded to the sample collection tube. In this way, the secondary reagent may be added again after sample extraction, which expands range of application of the sample extraction device. (Generally the secondary reagent is a reagent not suitable for direct transfer into the sample extraction device, such as a reagent that requires for low-temperature preservation. The present disclosure does not limit the secondary reagent that can be added).

The sample extraction device of the present disclosure is described in detail below through another specific example.

As shown in FIG. 11 and FIG. 12, the sample extraction device of the present disclosure comprises a plurality of chambers S arranged on the pedestal 10, wherein the chambers S comprise a plurality of first chambers S1 and two mixing chambers S2. The plurality of first chambers S1 comprise a sample suction tube S11, a lysis solution chamber S12, an empty chamber S13, at least one washing solution chamber such as a first washing solution chamber S14 and a second washing solution chamber S15, and an eluent chamber S16. The two mixing chambers S2 comprise a first mixing chamber S21 and a second mixing chamber S22. The sample suction tube S11, the lysis solution chamber S12, the empty chamber S13, the at least one washing solution chamber, the eluent chamber S16, the first mixing chamber S21 and the second mixing chamber S22 are sequentially arranged on the pedestal 10. The pedestal 10 is internally provided with a first passage L1 for communicating the two mixing chambers S2, and a sample extraction chamber R1 is arranged in the first passage L1. The pedestal 10 is further internally provided with a second passage L2 for transfer and export of a liquid. Connection or disconnection between the two mixing chambers S2 and the second passage L2 is controlled by the valve 20.

As a preferred solution, the sample extraction device of the present disclosure further includes a sealant chamber S17, which is internally provided with a sealing reagent selected from at least one of mineral oil, silicone oil, fluoroalkane oil, vegetable oil, and liquid paraffin. The sample suction tube S11, the lysis solution chamber S12, the empty chamber S13, the at least one washing solution chamber, the first mixing chamber S21, the second mixing chamber S22, the sealant chamber S17, and the eluent chamber S16 are sequentially arranged on the pedestal 10.

As another preferred solution, the sample extraction device of the present disclosure also includes a purge chamber S18. The sample suction tube S11, the lysis solution chamber S12, the empty chamber S13, the at least one washing solution chamber, the first mixing chamber S21, the second mixing chamber S22, the eluent chamber S16, and the purge chamber S18 are sequentially arranged on the pedestal 10.

As another preferred solution, the sample extraction device of the present disclosure further includes a sealant chamber S17 and a purge chamber S18. The sample suction tube S11, the lysis solution chamber S12, the empty chamber S13, the at least one washing solution chamber, the first mixing chamber S21, the second mixing chamber S22, the sealant chamber S17, the eluent chamber S16, and the purge chamber S18 are sequentially arranged on the pedestal 10.

Please also refer to FIG. 10, the front face of the pedestal 10 is provided with the first passage L1, a third channel L23, a fourth channel L24, the gas exhaust channel L6, and the second sample loading runner L8. The back face of the pedestal 10 is provided with a fifth channel L25, the liquid outlet channel L4, and the sample quantification channel L5.

The first passage L1 is connected to the first mixing chamber S21 and the second mixing chamber S22. The first passage L1 is internally provided with a sample extraction chamber R1. The first mixing chamber S21 and the second mixing chamber S22 are arranged with a preset interval to attach an external heating device. A second cavity R3 (not shown in the figure) is arranged below the sample extraction chamber R1, and a permanent magnet may be placed in the second cavity R3 to adsorb the magnetic beads.

The third channel L23 is controlled by a valve 20 respectively to connect the first washing solution chamber S14, the second washing solution chamber S15, and the first mixing chamber S21. The fourth channel L24 is controlled by a valve 20 respectively to connect the eluent chamber S16 and the second mixing chamber S22. The fifth channel L25 is controlled by a valve 20 respectively to connect the sample suction tube S11, the lysis solution chamber S12, the empty chamber S13, and the first mixing chamber S21.

A sample collection tube (indicated as C in the figure) is arranged at the outlet of the liquid outlet channel L4, and the liquid outlet channel L4 is controlled by a valve 20 respectively to connect the sample quantification channel L5 to the sample collection tube.

The sample quantification channel L5 is controlled by a valve 20 respectively to connect the second mixing chamber S22, the sealant chamber S17, and the purge chamber S18. The sample quantification channel L5 is internally provided with a quantification chamber R4, which is positioned in a passage part between the sealant chamber S17 and the purge chamber S18 connected under the control of a valve 20.

The gas exhaust channel L6 is connected to the purge chamber S18 and the sample collection tube under the control of a valve 20.

For the steps for nucleic acid extraction using the sample extraction device in embodiments of the present disclosure, one may refer to the relevant section of Example 1. It should be noted that in the present embodiment, the gas exhaust channel L6 is used to discharge the gas in the sample collection tube when exporting the sample, and to discharge the gas in the sample collection tube when the secondary reagent is added through the second sample loading tube S19. Reference may be made to specific technical details of the Examples for other similar sections between the present Example and Example 1, which are not described herein again.

The foregoing description is only explanation of the preferred embodiments of the present disclosure and the applied technical principles. It should be appreciated by those skilled in the art that the scope of the present invention is not limited to the technical solutions formed by the particular combinations of the above technical features. The scope should also cover other technical solutions formed by any combinations of the above technical features or equivalent features thereof without departing from the concept of the invention, such as, technical solutions formed by replacing the features as of the present disclosure with (but not limited to), technical features with similar functions.

## Claims

1. A sample extraction device comprising a plurality of chambers (S) arranged on a pedestal (10),
wherein the chambers (S) comprise a plurality of first chambers (S1) and two mixing chambers (S2);
the pedestal (10) is internally provided with a first passage (L1) for communicating the two mixing chambers (S2), and a sample extraction chamber (R1) is arranged in the first passage (L1); and
the pedestal (10) is further internally provided with a second passage (L2) for transfer and export of a liquid, and connection or disconnection between the two mixing chambers (S2) and the second passage (L2) is controlled by a valve (20).

2. A sample extraction device comprising a plurality of chambers (S) arranged on a pedestal (10),
wherein the chambers (S) comprise a plurality of first chambers (S1) and two mixing chambers (S2); the plurality of first chambers (S1) comprise: a sample suction tube (S11), a lysis solution chamber (S12), an empty chamber (S13), at least one washing solution chamber, and an eluent chamber (S16); and the two mixing chambers (S2) comprise a first mixing chamber (S21) and a second mixing chamber (S22);
wherein the sample suction tube (S11), the lysis solution chamber (S12), the empty chamber (S13), the at least one washing solution chamber, the eluent chamber (S16), the first mixing chamber (S21) and the second mixing chamber (S22) are sequentially arranged on the pedestal (10);
the pedestal (10) is internally provided with a first passage (L1) for communicating the two mixing chambers (S2), and a sample extraction chamber (R1) is arranged in the first passage (L1); and
the pedestal (10) is further internally provided with a second passage (L2) for transfer and export of a liquid, and connection or disconnection between the two mixing chambers (S2) and the second passage (L2) is controlled by a valve (20).

3. The sample extraction device according to claim 2, wherein the plurality of first chambers (S1) further comprise a sealant chamber (S17) and/or a purge chamber (S18);
the sample suction tube (S11), the lysis solution chamber (S12), the empty chamber (S13), the at least one washing solution chamber, the eluent chamber (S16), the sealant chamber (S17), the first mixing chamber (S21), the second mixing chamber (S22), and the purge chamber (S18) are sequentially arranged on the pedestal (10); or
the sample suction tube (S11), the lysis solution chamber (S12), the empty chamber (S13), the at least one washing solution chamber, the eluent chamber (S16), the first mixing chamber (S21), the second mixing chamber (S22), and the purge chamber (S18) are sequentially arranged on the pedestal (10); or
the sample suction tube (S11), the lysis solution chamber (S12), the empty chamber (S13), the at least one washing solution chamber, the eluent chamber (S16), the sealant chamber (S17), the first mixing chamber (S21), and the second mixing chamber (S22) are sequentially arranged on the pedestal (10).

4. The sample extraction device according to claim 2, wherein the plurality of first chambers (S1) further comprise a sealant chamber (S17) and/or a purge chamber (S18);
the sample suction tube (S11), the lysis solution chamber (S12), the empty chamber (S13), the at least one washing solution chamber, the first mixing chamber (S21), the second mixing chamber (S22), the sealant chamber (S17), the eluent chamber (S16), and the purge chamber (S18) are sequentially arranged on the pedestal (10); or
the sample suction tube (S11), the lysis solution chamber (S12), the empty chamber (S13), the at least one washing solution chamber, the first mixing chamber (S21), the second mixing chamber (S22), the sealant chamber (S17), and the eluent chamber (S16) are sequentially arranged on the pedestal (10); or
the sample suction tube (S11), the lysis solution chamber (S12), the empty chamber (S13), the at least one washing solution chamber, the first mixing chamber (S21), the second mixing chamber (S22), the eluent chamber (S16), and the purge chamber (S18) are sequentially arranged on the pedestal (10).

5. The sample extraction device according to any one of claims 1 to 4, further comprising a valve assembly configured to control connection or disconnection between the second passage (L2) and the plurality of first chambers (S 1).

6. The sample extraction device according to claim 5, wherein at least one of the plurality of first chambers (S 1) is preloaded with a reagent required for sample extraction.

7. The sample extraction device according to claim 5, wherein the plurality of chambers (S) are sealed by a sealing element selected from a membrane, a piston, or a piston rod.

8. The sample extraction device according to claim 5, wherein the two mixing chambers (S2) are arranged with a preset interval; and/or, a second cavity (R3) is provided below the sample extraction chamber (R1).

9. The sample extraction device according to claim 5, wherein a magnetic bead reagent is prepackaged in the sample extraction chamber (R1).

10. The sample extraction device according to claim 5, further comprising:
a quantitation chamber (R4) positioned within the second passage (L2).

11. The sample extraction device according to claim 5, wherein the valve assembly is consisted of a plurality of valves (20); and the second passage (L2) has branches extending towards the first chambers (S1) and the mixing chambers (S2), and connection or disconnection between each of the branches and a first chamber (S1) or a mixing chamber (S2) is independently controlled by one of the plurality of valves (20).

12. The sample extraction device according to claim 11, wherein the valve (20) is a rotating member, and the plurality of valves (20) are arranged linearly at a bottom of the pedestal (10).

13. The sample extraction device according to claim 12, wherein the bottom of the pedestal (10) is provided with a plurality of first cavities (R2), and each of the plurality of first cavities (R2) is provided with a valve (20) having an adapter channel; and when the valve (20) is placed in the first cavity (R2), one end of the adapter channel is communicated with a chamber (S), and another end of the adapter channel is communicated with a second passage (L2) under an external force.

14. The sample extraction device according to claim 13, further comprising a sealing plug (40) arranged in the first cavity (R2), wherein the sealing plug (40) is respectively communicated with a chamber (S) and a second passage (L2), and the adapter channel is placed in the sealing plug (40).

15. The sample extraction device according to claim 14, wherein the sealing plug (40) is a column structure, a through hole (41) is provided in a center of the sealing plug (40), and at least one open hole (42) is provided on a side wall of the sealing plug (40).

16. The sample extraction device according to claim 13, wherein the valve (20) comprises a valve handle (21) and a valve shaft (22); the valve handle (21) is movably connected to the pedestal (10), and the adapter channel is arranged inside the valve shaft (22).

17. The sample extraction device according to claim 16, wherein the valve handle (21) and valve shaft (22) are of an integrated or detachable structure.

18. The sample extraction device according to claim 16, wherein one end of the valve handle (21) is provided with a limiting slot.

19. The sample extraction device according to claim 5, further comprising:
a first sample loading tube (S3) and a first sample loading runner (L3) arranged on the pedestal (10), wherein the first sample loading runner (L3) is controlled by a valve (20) to connect the first sample loading tube (S3) to one of the first chambers (S1), and/or one of the first chambers (S1) is internally provided with a first push-pull piston rod (31); or
a branch tube (S4) is communicated with at least one of the first chambers (S1), and/or the at least one of the first chambers (S1) is internally provided with a third push-pull piston rod (33), a top of the third push-pull piston rod (33) is provided with a sealing ring, wherein when the third push-pull piston rod (33) is pushed to a bottom, the sealing ring is positioned above the branch tube (S4).

20. The sample extraction device according to claim 19, wherein the at least one of the first chambers (S1) is a sample suction tube (S1 1).

21. The sample extraction device according to claim 5, wherein
the second passage (L2) comprises a liquid outlet channel (L4);
the sample extraction device further comprises:
a second sample loading tube (S19), a second sample loading runner (L8) and a sample collection tube arranged on the pedestal (10); wherein the sample collection tube is arranged at a liquid outlet of the liquid outlet channel (L4); and
the second sample loading runner (L8) is controlled by a valve (20) to connect the second sample loading tube (S19) to the liquid outlet channel (L4); wherein the second sample loading tube (S19) has a branch tube (S4).

22. The sample extraction device according to claim 21, wherein the second sample loading tube (S19) is internally provided with a third push-pull piston rod (33), a top of the third push-pull piston rod (33) is provided with a sealing ring, wherein when the third push-pull piston rod (33) is pushed to a bottom, the sealing ring is positioned above the branch tube (S4).

23. The sample extraction device according to claim 5, further comprising:
a sample collection tube arranged on the pedestal (10); and
a gas exhaust channel (L6) controlled by a valve (20) to connect one of the plurality of first chambers (S1) to the sample collection tube.

24. The sample extraction device according to claim 5, wherein the pedestal (10) comprises a pedestal body (11) and an outer protective plate (12) positioned on a front face and a back face of the pedestal body (11); at least one groove (111) is provided on the front face and/or back face of the pedestal body (11); and the groove (111) and the outer protective plate (12) form the first passage (L1) and the second passage (L2).

25. The sample extraction device according to claim 3, further comprising:
a sample collection tube arranged on the pedestal (10); wherein
the second passage (L2) comprises:
a first channel (L21) controlled by a valve (20) respectively to connect the at least one washing solution chamber, the eluent chamber (S16), and the first mixing chamber (S21);
a second channel (L22) controlled by a valve (20) respectively to connect the sample suction tube (S11), the lysis solution chamber (S12), the empty chamber (S13), and the first mixing chamber (S21);
a sample quantification channel (L5) controlled by a valve (20) respectively to connect the sealant chamber (S17) and/or the purge chamber (S18) and the second mixing chamber (S22), wherein the sample quantification channel (L5) is internally provided with a quantification chamber (R4);
a liquid outlet channel (L4) controlled by a valve (20) respectively to connect the sample quantification channel (L5) and the sample collection tube; and
a gas exhaust channel (L6) controlled by a valve (20) to connect the eluent chamber (S16) and the sample collection tube.

26. The sample extraction device according to claim 3, further comprising:
a sample collection tube arranged on the pedestal (10); wherein
the second passage (L2) comprises:
a third channel (L23) controlled by a valve (20) respectively to connect the at least one washing solution chamber and the first mixing chamber (S21);
a fourth channel (L24) controlled by a valve (20) respectively to connect the eluent chamber (S16) and the second mixing chamber (S22);
a fifth channel (L25) controlled by a valve (20) respectively to connect the sample suction tube (S11), the lysis solution chamber (S12), the empty chamber (S13), and the first mixing chamber (S21);
a sample quantification channel (L5) controlled by a valve (20) respectively to connect the second mixing chamber (S22), the sealant chamber (S17) and/or the purge chamber (S18), wherein the sample quantification channel (L5) is internally provided with a quantification chamber (R4);
a liquid outlet channel (L4) controlled by a valve (20) respectively to connect the sample quantification channel (L5) and the sample collection tube; and
a gas exhaust channel (L6) controlled by a valve (20) to connect the purge chamber (S18) and the sample collection tube.
